# EUROPEAN PATENT APPLICATION

(11) **EP 4 850 588 A1**
(43) Date of publication of application: **09.12.2026**
(21) Application number: 26182850.3
(22) Date of filing: 03.06.2026
(51) Int. Cl.: A61B 1/04, A61B 1/00, A61B 1/06, A61B 1/045, G01N 21/64, A61B 5/00, H04N 25/10

(54) **SYSTEMS AND METHODS FOR PROVIDING MEDICAL FLUORESCENCE IMAGING USING PIXEL-SPECIFIC EXPOSURE SCHEME**

(30) Priority: 04.06.2025 US 202563817873 P
(71) Applicant: Stryker Corporation, Portage, MI 49002-9711 (US)
(72) Inventor: ANDRÉ, Marc, Portage, 49002 (US)
(74) Representative: V.O.

(57) **Abstract**

The present disclosure relates to techniques for imaging tissue of a subject. An exemplary method comprises during a single frame acquisition period: exposing a group of fluorescence imaging pixels of an image sensor to accumulate charge for generating a fluorescence image during a fluorescence exposure duration of the single frame acquisition period and exposing a group of visible-light imaging pixels of the image sensor to accumulate charge for a visible-light exposure duration of the single frame acquisition period. The method further comprises reading the accumulated charge at the group of fluorescence imaging pixels and the accumulated charge at the group of visible-light imaging pixels of the image sensor and generating the image of the tissue.

## Description

### CROSS-REFERENCE TO RELATED APPLICATIONS

This application claims the benefit of U.S. Provisional Application No. 63/817,873, filed June 4, 2025, the entire contents of which are incorporated herein by reference.

### FIELD

The present disclosure relates generally to medical imaging, and more specifically to techniques for providing medical fluorescence imaging (e.g., open-field surgery fluorescence visualization) using pixel-specific exposure scheme.

### BACKGROUND

Medical imaging systems enable a surgeon to view a surgical site in open-field procedures and endoscopic procedures. Endoscopy allows internal features of the body of a patient to be viewed without the use of traditional, fully invasive surgery. Endoscopic tools can be used with the endoscopic imaging systems to enable non-invasive surgery at the site. An endoscopic imaging system typically includes a camera head connected to a camera control unit (CCU) that processes input image data received from the image sensor of the camera and outputs the image data for display. The CCU may control an illuminator or illumination source that generates illumination light provided to the imaged scene.

Medical imaging systems (e.g., endoscopic imaging systems, open-field imaging systems) may include both a visible-light illumination source (also referred to as white-light illumination source) and a fluorescence excitation illumination source. The visible-light illumination source and the fluorescence excitation illumination source can be used to illuminate a tissue of a subject to obtain different types of image data (e.g., fluorescence image frames, white-light image frames, blended image frames). In particular, the fluorescence excitation illumination source, such as an infrared light, can provide illumination to the imaged tissue to excite the fluorophores (or fluorochromes) to produce fluorescence emission.

In existing medical imaging systems, an image sensor may be configured to be exposed to acquire different types of imaging data in different frame periods. For example, the image sensor may be configured to expose the pixels of the image sensor while a tissue is illuminated with a visible-light illumination source and a fluorescence excitation illumination source in an alternating manner, such that the system can acquire visible-light imaging data in one frame acquisition period, fluorescence imaging data in the next frame period, and so on. The visible-light imaging data and/or the fluorescence imaging data can be used to generate a composite image or be used for image adjustment (e.g., using the visible-light imaging data for auto gain adjustment of the fluorescence image).

However, designating an entire frame acquisition period for acquiring a single type of imaging data have several drawbacks. For example, fluorescence signals tend to be relatively weaker and the image sensor can benefit from longer exposure time to acquire high-quality fluorescence data. On the other hand, visible-light imaging data may not require as much exposure time. The visible-light signal tends to be stronger and the visible-light imaging data may only be used as a background image or for image adjustment. However, in existing medical imaging systems, the fluorescence imaging data and the visible-light imaging data have the same amount of time allocated to frame acquisition (e.g., one single frame acquisition period), thus resulting in under-utilization of the total available exposure time and poor fluorescence image quality.

Thus, techniques for better utilization of the image sensor to capture higher quality fluorescence imaging data without compromising frame rate are desirable.

### SUMMARY

Examples of the present disclosure include systems, devices, methods, non-transitory storage media, computer program products, and apparatuses for generating an image of a tissue. Rather than acquiring a single type of imaging data per frame acquisition period, an exemplary system may be configured to, within a frame acquisition period, expose different subsets of pixels of the image sensor to acquire different types of imaging data. For example, the system may, during a single frame acquisition period, expose a group of fluorescence imaging pixels of the image sensor to accumulate charge during a fluorescence exposure duration of the single frame acquisition period and expose a group of visible-light imaging pixels of the image sensor to accumulate charge for a visible-light exposure duration of the single frame acquisition period. The system can read the accumulated charge at the group of fluorescence imaging pixels and the accumulated charge at the group of visible-light imaging pixels of the image sensor to generate an image of the tissue from the read accumulated charges. In particular, the fluorescence exposure duration may be longer than the visible-light exposure duration, increasing fluorescence imaging sensitivity as a larger proportion of the frame acquisition period is devoted to fluorescence exposure.

In the present disclosure, the acquired fluorescent image may be a brightness adjusted fluorescence image. The image may alternatively be a composite image, such as an overlay image based on the fluorescence image and the visible-light image. In some examples, the pixels of the image sensor are divided into multiple groups, each for acquiring a different type of imaging data (e.g., different types of fluorescence imaging data corresponding to different fluorophores, visible-light imaging data, ambient imaging data). Depending on the desirable image resolution or quality, the system can designate a larger or smaller number of pixels for a particular type of imaging data. For example, in contrast fluorescence imaging mode, the system can allow for three pixels of a Bayer 2x2 array to be used for a longer fluorescence exposure and one pixel for visible-light exposure (e.g., for auto gain) or ambient light exposure (e.g., for room light subtraction). In fluorescence overlay imaging mode, R,G,B pixels in a Bayer RGGB 2x2 array can be exposed to generate a visible-light image while one G pixel is used for a longer fluorescence exposure, all within one frame acquisition period.

Examples of the present disclosure can provide several technical advantages. In a conventional imaging system, the system alternates between a visible-light image frame acquisition period and a fluorescence image frame acquisition period, with all pixels of the image sensor exposed together. This is not ideal because the image sensor is allocated for the same amount of time (i.e., a single frame acquisition period) for both types of imaging data, even though the fluorescence imaging data can benefit from longer exposure time and the visible-light imaging data does not need as much exposure time. In contrast, in examples of the present disclosure, the system can output frames at the same frame rate but allow for longer exposure time for acquiring fluorescence imaging data and leave no time wasted/unused during the frame period. Further, more of the pixels of the image sensor can be configured to be exposed for a longer duration to obtain fluorescence imaging data, resulting in a richer and higher-resolution fluorescence image. Thus, the pixel-specific exposure scheme can result in higher-quality fluorescence imaging data (e.g., better signal-to-noise ratio) without compromising frame rate and while still acquiring visible-light imaging data. The pixel-specific exposure scheme enables improved fluorescence imaging sensitivity and/or frame rate without requiring multiple image sensors. Further, the system places less burden on the cable, allowing higher frame rate and/or lower bandwidth on the cable and thus enabling a cheaper camera. Controlling pixels with independent timing allows fluorescence acquisition or High Dynamic Range (HDR) acquisition without increasing the frame rate. This also allows a sensor with limited framerate to perform, for example, image acquisition involving multiple different fluorophores.

An exemplary method for generating an image of a tissue comprises: during a single frame acquisition period: exposing a group of fluorescence imaging pixels of an image sensor to accumulate charge for generating a fluorescence image during a fluorescence exposure duration of the single frame acquisition period; exposing a group of visible-light imaging pixels of the image sensor to accumulate charge for a visible-light exposure duration of the single frame acquisition period; reading the accumulated charge at the group of fluorescence imaging pixels and the accumulated charge at the group of visible-light imaging pixels of the image sensor; and generating the image of the tissue from the read accumulated charge at the group of fluorescence imaging pixels and the read accumulated charge at the group of visible-light imaging pixels of the image sensor.

In some examples, the tissue is illuminated with a fluorescence excitation illumination source during the fluorescence exposure duration. In some examples, the tissue is illuminated with a visible-light illumination source during the visible-light exposure duration.

In some examples, the image comprises a brightness adjusted fluorescence image. In some examples, generating the brightness adjusted fluorescence image comprises: determining a brightness level based on the read accumulated charge at the group of visible-light imaging pixels of the image sensor; and determining a gain setting based on the brightness level.

In some examples, the method further comprises generating a visible-light image of the tissue from the read accumulated charge at the group of bright-light imaging pixels of the image sensor; and generating an overlay image based on the fluorescence image and the visible-light image of the tissue.

In some examples, the fluorescence exposure duration and the visible-light exposure duration do not overlap. The fluorescence exposure duration and the visible-light exposure duration may be specified by one or more user inputs. In some examples, the fluorescence exposure duration is longer than the visible-light exposure duration.

In some examples, the image sensor comprises a plurality of pixels divided into a plurality of pixel arrays. In some examples, the group of fluorescence imaging pixels comprises one or more pixels from each pixel array of the plurality of pixel arrays, and the group of bright-light imaging pixels comprises one or more pixels from each pixel array of the plurality of pixel arrays. In some examples, each pixel array of the plurality of pixel arrays is a 2x2 pixel array. The group of fluorescence imaging pixels comprises 3 pixels from each 2x2 pixel array of the plurality of 2x2 pixel arrays, and the group of bright-light imaging pixels comprises one pixel from each 2x2 pixel array of the plurality of 2x2 pixel arrays.

In some examples, the tissue is illuminated with ambient illumination and not a bright-light illumination source during the visible-light exposure duration. In some examples, the tissue is illuminated with both ambient illumination and a bright-light illumination source during the visible-light exposure duration, the method further comprising: exposing, during the single frame acquisition period, a group of ambient imaging pixels of the image sensor to accumulate charge for an ambient light exposure duration while the tissue is not illuminated by the bright-light illumination source or the fluorescence excitation illumination source; reading the accumulated charge at the group of ambient imaging pixels of the image sensor; and determining an ambient brightness level based on the read accumulated charge at the group of ambient imaging pixels of the image sensor.

In some examples, the fluorescence excitation illumination source is a first fluorescence excitation illumination source corresponding to a first fluorophore, wherein the fluorescence exposure duration is a first fluorescence exposure duration, and wherein the group of fluorescence imaging pixels is a first group of fluorescence imaging pixels, the method further comprising: exposing, during the single frame acquisition period, a second group of fluorescence imaging pixels of the image sensor to accumulate charge for a second fluorescence exposure duration while the tissue is illuminated by a second fluorescence excitation illumination source corresponding to a second fluorophore; reading the accumulated charge at the second group of fluorescence imaging pixels of the image sensor; and generating a second fluorescence image based on the read accumulated charge at the second group of fluorescence imaging pixels of the image sensor.

In some examples, the image sensor is a Bayer sensor. In some examples, the image sensor is one of a plurality of image sensors of a multi-sensor imaging system. The image sensor may be a global shutter image sensor. The image sensor may be part of an endoscopic imager, a flexible and/or chip-on-tip scope, or an open field imager. The endoscopic imager can include an endoscope or scope coupled to a camera head. The endoscope may be pre-inserted into a lumen of a body of a patient prior to start of any of the methods described herein.

An exemplary system for generating an image of a tissue comprises an image sensor configured for performing any of the methods described herein.

It will be appreciated that any of the aspects and examples described hereinabove may be combined. Any examples provided in view of the method also apply to to the system.

### BRIEF DESCRIPTION OF THE FIGURES

The invention will now be described, by way of example only, with reference to the accompanying drawings, in which:
FIG. 1A is an illustration of an endoscopic camera system, according to some examples;
FIG. 1B is a diagram of a portion of the endoscopic camera system of FIG. 1A and a target object for imaging, according to some examples;
FIG. 2A illustrates a schematic view of a system for illumination and imaging in open-field surgeries, according to some examples;
FIG. 2B illustrates another exemplary system for illumination and imaging in open-field surgeries, according to some examples;
FIG. 3 is a block diagram of an imaging system, according to some examples;
FIG. 4 provides an exemplary method for imaging a tissue of a subject, in accordance with some examples;
FIG. 5 illustrates an exemplary pixel designation scheme for an image sensor for a given frame acquisition period, in accordance with some examples;
FIG. 6A illustrates an exposure timing diagram for the prior art imaging system, in accordance with some examples;
FIG. 6B illustrates an exposure timing diagram for an exemplary system, in accordance with some examples;
FIG. 7 illustrates another exemplary imaging system, in accordance with some examples;
FIG. 8 illustrates another exemplary imaging system, in accordance with some examples;
FIG. 9 illustrates an exemplary configuration of exposure time for each pixel in a 2-by-2 pixel array, in accordance with some examples.
FIG. 10 illustrates an exemplary electronic system, in accordance with some examples.

### DETAILED DESCRIPTION

Reference will now be made in detail to implementations and examples of various aspects and variations of systems and methods described herein. Although several exemplary variations of the systems and methods are described herein, other variations of the systems and methods may include aspects of the systems and methods described herein combined in any suitable manner having combinations of all or some of the aspects described. Examples will now be described more fully hereinafter with reference to the accompanying drawings; however, they may be embodied in different forms and should not be construed as limited to the examples set forth herein. Rather, these examples are provided so that this disclosure will be thorough and complete, and will fully convey exemplary implementations to those skilled in the art.

Examples of the present disclosure include systems, devices, methods, non-transitory storage media, and apparatuses for generating an image of a tissue. Rather than acquiring a single type of imaging data per frame acquisition period, an exemplary system may be configured to, within a frame acquisition period, expose different subsets of pixels of the image sensor to acquire different types of imaging data. For example, the system may, during a single frame acquisition period, expose a group of fluorescence imaging pixels of the image sensor to accumulate charge during a fluorescence exposure duration of the single frame acquisition period and expose a group of visible-light imaging pixels of the image sensor to accumulate charge for a visible-light exposure duration of the single frame acquisition period. The system can read the accumulated charge at the group of fluorescence imaging pixels and the accumulated charge at the group of visible-light imaging pixels of the image sensor to generate an image of the tissue from the read accumulated charges. In particular, the fluorescence exposure duration may be longer than the visible-light exposure duration, increasing fluorescence imaging sensitivity as a larger proportion of the frame acquisition period is devoted to fluorescence exposure.

The image may be a brightness adjusted fluorescence image. The image may alternatively be a composite image, such as an overlay image based on the fluorescence image and the visible-light image. In some examples, the pixels of the image sensor are divided into multiple groups, each for acquiring a different type of imaging data (e.g., different types of fluorescence imaging data corresponding to different fluorophores, visible-light imaging data, ambient imaging data). Depending on the desirable image resolution or quality, the system can designate a larger or smaller number of pixels for a particular type of imaging data. For example, in contrast fluorescence imaging mode, the system can allow for three pixels of a Bayer 2x2 array to be used for a longer fluorescence exposure and one pixel for visible-light exposure (e.g., for auto gain) or ambient light exposure (e.g., for room light subtraction). In fluorescence overlay imaging mode, R,G,B pixels in a Bayer RGGB 2x2 array can be exposed to generate a visible-light image while one G pixel is used for a longer fluorescence exposure, all within one frame acquisition period.

Examples of the present disclosure can provide several technical advantages. In a conventional imaging system, the system alternates between a visible-light image frame acquisition period and a fluorescence image frame acquisition period, with all pixels of the image sensor exposed together. This is not ideal because the image sensor is allocated for the same amount of time (i.e., a single frame acquisition period) for both types of imaging data, even though the fluorescence imaging data can benefit from longer exposure time and the visible-light imaging data does not need as much time. In contrast, in examples of the present disclosure, the system can output frames at the same frame rate but allow for longer exposure time for acquiring fluorescence imaging data and leave no time wasted/unused during the frame period. A subset of pixels of the image sensor can be configured to be exposed for a longer duration to obtain fluorescence imaging data, resulting in a richer and yield a fluorescence image with an improved signal-to-noise ratio. Thus, the pixel-specific exposure scheme can result in higher-quality fluorescence imaging data (e.g., better signal-to-noise ratio) without compromising frame rate and while still acquiring visible-light imaging data. The pixel-specific exposure scheme enables improved fluorescence imaging sensitivity and/or frame rate without requiring multiple image sensors. Further, the system places less burden on the cable, allowing higher frame rate and/or lower bandwidth on cable and thus requiring a cheaper camera. Controlling pixels with independent timing allows fluorescence acquisition or HDR without increasing the frame rate. This also allows a sensor with limited framerate to perform, for example, image acquisition involving multiple different fluorophores.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

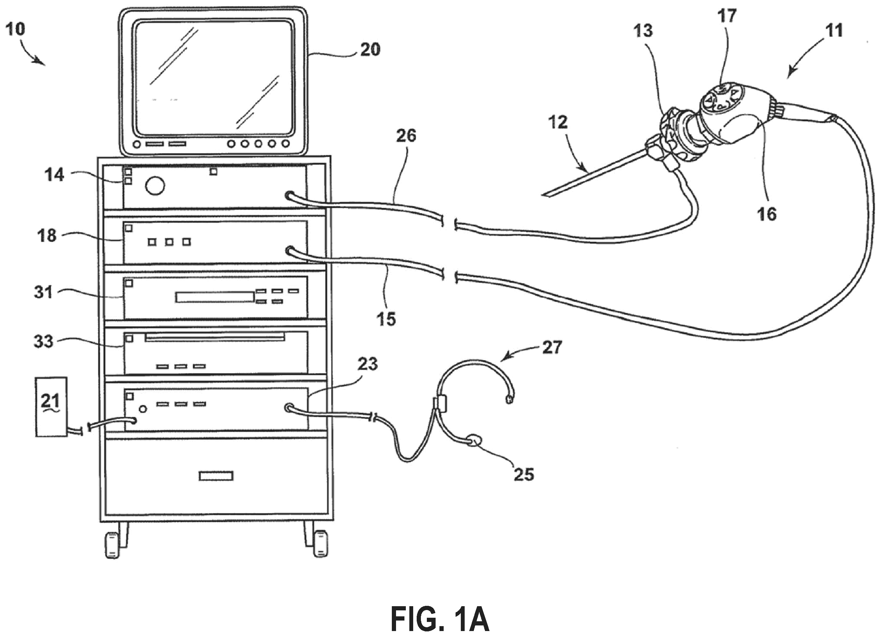

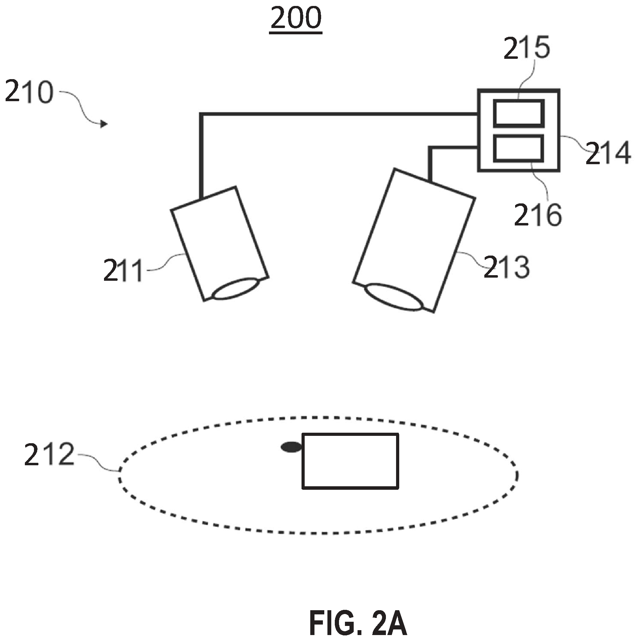

Examples of the present disclosure may be incorporated into any medical imaging systems, including imaging systems used in minimally invasive surgeries (e.g., FIGS. 1A-B) and open field imaging systems (e.g., FIG. 2A). FIG. 1A shows an example of an endoscopic imaging system 10, which includes a scope assembly 11 which may be utilized in endoscopic procedures. The scope assembly 11 incorporates an endoscope or scope 12 which is coupled to a camera head 16 by a coupler 13 located at the distal end of the camera head 16. The endoscope 12 may be pre-inserted into a lumen of a body of a patient prior to start of any of the methods described herein. Light is provided to the scope by a light source 14 via a light guide 26, such as a fiber optic cable. The camera head 16 is coupled to a camera control unit (CCU) 18 by an electrical cable 15. The CCU 18 is connected to, and communicates with, the light source 14. Operation of the camera 16 is controlled, in part, by the CCU 18. The cable 15 conveys video image and/or still image data from the camera head 16 to the CCU 18 and may convey various control signals bi-directionally between the camera head 16 and the CCU 18.

A control or switch arrangement 17 may be provided on the camera head 16 for allowing a user to manually control various functions of the system 10, which may include switching from one imaging mode to another, as discussed further below. Voice commands may be input into a microphone 25 mounted on a headset 27 worn by the practitioner and coupled to the voice-control unit 23. A hand-held control device 29, such as a tablet with a touch screen user interface or a PDA, may be coupled to the voice control unit 23 as a further control interface. In the illustrated example, a recorder 31 and a printer 33 are also coupled to the CCU 18. Additional devices, such as an image capture and archiving device, may be included in the system 10 and coupled to the CCU 18. Video image data acquired by the camera head 16 and processed by the CCU 18 is converted to images, which can be displayed on a monitor 20, recorded by recorder 31, and/or used to generate static images, hard copies of which can be produced by the printer 33.

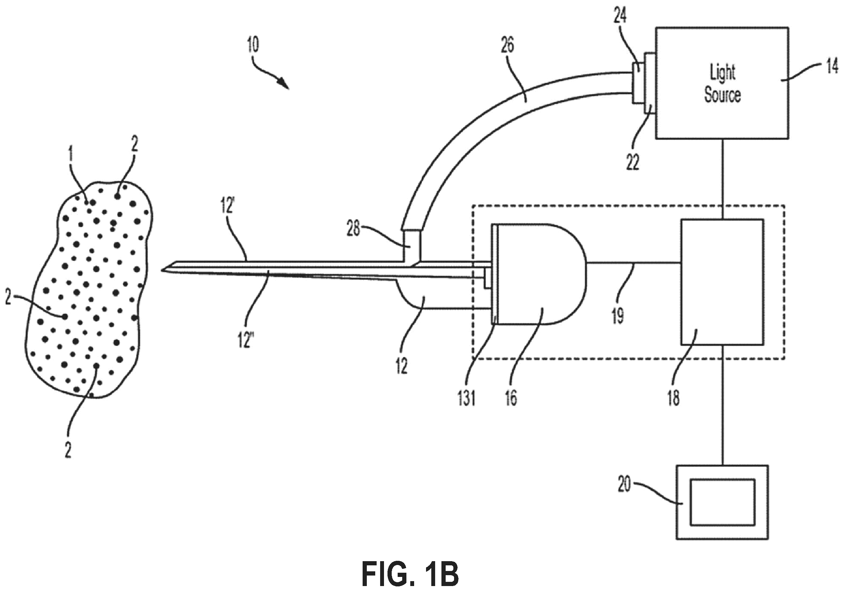

FIG. 1B shows an example of a portion of the endoscopic system 10 being used to illuminate and receive light from an object 1, such as a surgical site of a patient. The object 1 may include fluorescent markers 2, for example, as a result of the patient being administered a fluorescence imaging agent. The fluorescent markers 2 may comprise, for example, indocyanine green (ICG).

The light source 14 can generate visible illumination light (such as any combination of red, green, and blue light) for generating visible (e.g., white light) images of the target object 1 and, in some examples, can also produce fluorescence excitation illumination light for exciting the fluorescent markers 2 in the target object for generating fluorescence images. In some examples, the light source 14 can produce fluorescence excitation illumination light for exciting autofluorescence in the target object for generating fluorescence images, additionally or alternatively to light for exciting the fluorescent markers. Illumination light is transmitted to and through an optic lens system 22 which focuses light onto a light pipe 24. The light pipe 24 may create a homogeneous light, which is then transmitted to the fiber optic light guide 26. The light guide 26 may include multiple optic fibers and is connected to a light post 28, which is part of the endoscope 12. The endoscope 12 includes an illumination pathway 12' and an optical channel pathway 12".

The endoscope 12 may include a notch filter 131 that allows some or all (preferably, at least 80%) of fluorescence emission light *(e.g.,* in a wavelength range of 830 nm to 870 nm) emitted by fluorescence markers 2 in the target object 1 to pass therethrough and that allows some or all (preferably, at least 80%) of visible light *(e.g.,* in the wavelength range of 400 nm to 700 nm), such as visible illumination light reflected by the target object 1, to pass therethrough, but that blocks substantially all of the fluorescence excitation light *(e.g.,* infrared light having an infrared wavelength such as 780nm, 808 nm, or the like) that is used to excite fluorescence emission from the fluorescent marker 2 in the target object 1. The notch filter 131 may have an optical density of OD5 or higher. In some examples, the notch filter 131 can be located anywhere between the endoscope and the image sensor.

FIG. 2A illustrates an exemplary open field imaging system in accordance with some examples. FIG. 2A illustrates a schematic view of an illumination and imaging system 210 that can be used in open field surgical procedures. As may be seen therein, the system 210 may include an illumination module 211, an imaging module 213, and a video processor/illuminator (VPI) 214. The VPI 214 may include an illumination source 215 to provide illumination to the illumination module 211 and a processor assembly 216 to send control signals and to receive data about light detected by the imaging module 213 from a target 212 illuminated by light output by the illumination module 211. In one variation, the video processor/illuminator 214 may comprise a separately housed illumination source 215 and the processor assembly 216. In one variation, the video processor/illuminator 214 may comprise the processor assembly 216 while one or more illumination sources 215 are separately contained within the housing of the illumination module 211. The illumination source 215 may output light at different waveband regions, e.g., white (RGB) light, excitation light to induce fluorescence in the target 212, a combination thereof, and so forth, depending on characteristics to be examined and the material of the target 212. Light at different wavebands may be output by the illumination source 215 simultaneously, sequentially, or both. The illumination and imaging system 210 may be used, for example, to facilitate medical *(e.g.,* surgical) decision-making, *e.g.,* during a surgical procedure. The target 212 may include fluorescent markers, for example, as a result of the patient being administered a fluorescence imaging agent. The fluorescent markers may comprise, for example, indocyanine green (ICG). The target 212 may be a topographically complex target, *e.g.,* a biological material including tissue, an anatomical structure, other objects with contours and shapes resulting in shadowing when illuminated, and so forth. The VPI 214 may record, process, display, and so forth, the resulting images and associated information.

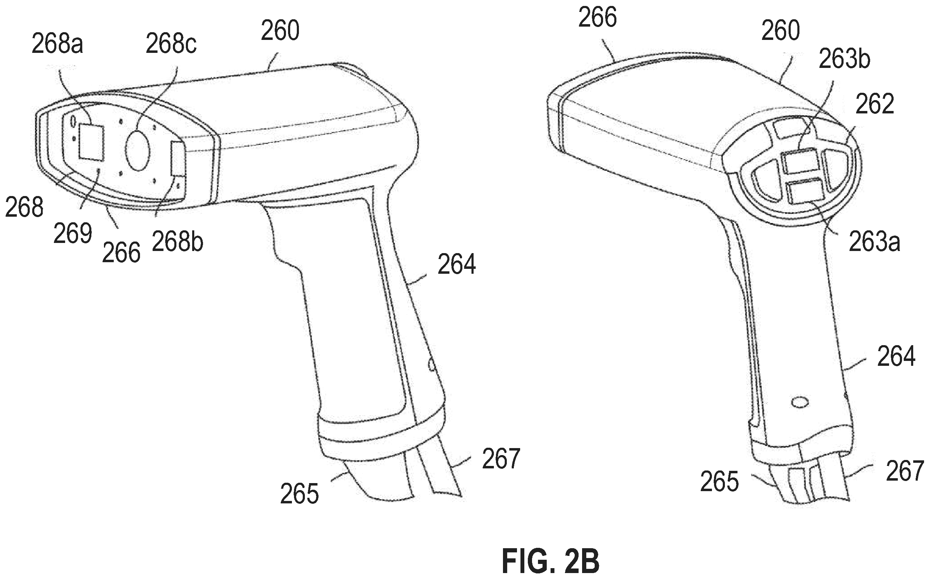

FIG. 2B illustrates an additional example of an open field surgical imaging system or a portion thereof. With reference to FIG. 2B, an ergonomic enclosure 260 can be designed to be held in a pistol-style grip. The enclosure 260 may include a control surface 262, a grip 264, a window frame 268 and a nosepiece 266. The ergonomic enclosure 260 is connectable to a VPI box (not depicted) via a light guide cable 267, through which the light is provided to one or more illumination ports (not depicted), and a data cable 265 that transmits power, sensor data, and any other (non-light) connections.

The control surface 262 includes focus buttons 263a and 263b that control the focus actuation assembly. Other buttons on the control surface 262 may be programmable and may be used for various other functions, excitation laser power on/off, display mode selection, white light imaging white balance, saving a screenshot, and so forth. Alternatively or additionally to the focus buttons, a proximity sensor may be provided on the enclosure and may be employed to automatically adjust the focus actuation assembly.

Enclosure 260 may be operated by a single hand in a pistol-grip style orientation. In various other examples, the enclosure 260 may be supported on a support (e.g., a movable support). In some examples, enclosure 260 may be used in concert with a drape. Window frame 268 may include windows 268a and 268b, corresponding to two lens modules, as well as window 268c, which serves as an input window for light from the target to be incident on the image sensor. Window frame 268 may also include one or more windows 269 for sensors provided behind a plate.

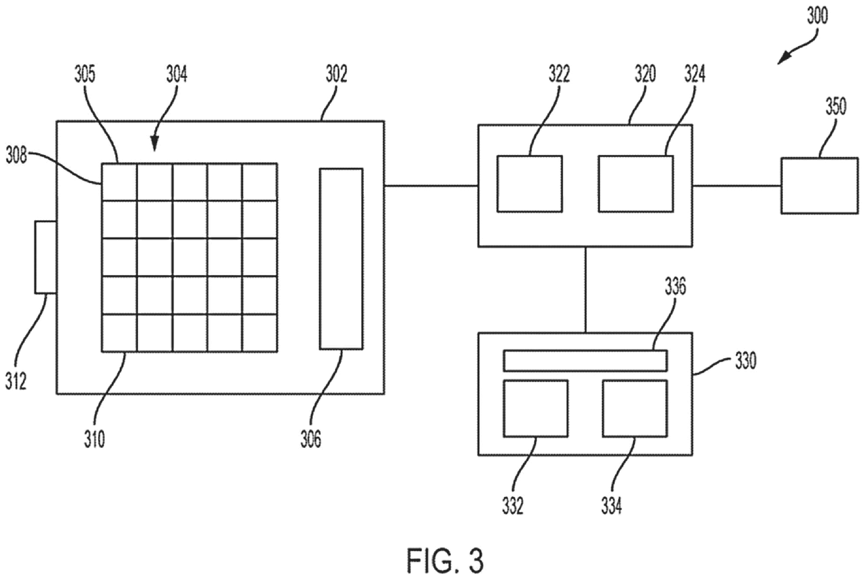

FIG. 3 schematically illustrates an exemplary imaging system 300 that employs an electronic imager 302 to generate images (*e.g.,* still and/or video) of a target object, such as a target tissue of a patient, according to some examples. The imager 302 may be a global shutter imager (*e.g.,* CCD sensors, CMOS sensors). System 300 may be used, for example, for the endoscopic imaging system 10 of FIG. 1A. The imager 302 includes a CMOS sensor 304 having an array of pixels 305 arranged in rows of pixels 308 and columns of pixels 310. The imager 302 may include control components 306 that control the signals generated by the CMOS sensor 304. Examples of control components include gain circuitry for generating a multi-bit signal indicative of light incident on each pixel of the sensor 304, one or more analog-to-digital converters, one or more line drivers to act as a buffer and provide driving power for the sensor 304, row circuitry, and timing circuitry. A timing circuit may include components such as a bias circuit, a clock/timing generation circuit, and/or an oscillator. Row circuitry may enable one or more processing and/or operational tasks such as addressing rows of pixels 308, addressing columns of pixels 310, resetting charge on rows of pixels 308, enabling exposure of pixels 305, decoding signals, amplifying signals, analog-to-digital signal conversion, applying timing, readout, and reset signals, and other suitable processes or tasks. Imager 302 may also include a shutter 312 that may be used, for example, to control exposure of the image sensor 304 and/or to control an amount of light received at the image sensor 304.

One or more control components may be integrated into the same integrated circuit in which the sensor 304 is integrated or may be discrete components. The imager 302 may be incorporated into an imaging head, such as camera head 16 of system 10.

One or more control components 306, such as row circuitry and a timing circuit, may be electrically connected to an imaging controller 320, such as camera control unit 18 of system 10; in some examples, they may be part of each pixel or groups of pixels. The imaging controller 320 may include one or more processors 322 and memory 324. The imaging controller 320 receives imager row readouts and may control readout timings and other imager operations, including mechanical shutter operation. The imaging controller 320 may generate image frames, such as video frames from the row and/or column readouts from the imager 302. Generated frames may be provided to a display 350 for display to a user, such as a surgeon.

The system 300 in this example includes a light source 330 for illuminating a target scene. The light source 330 is controlled by the imaging controller 320. The imaging controller 320 may determine the type of illumination provided by the light source 330 *(e.g.,* white light, fluorescence excitation light, or both), the intensity of the illumination provided by the light source 330, and or the on/off times of illumination in synchronization with image sensor shutter operation. The light source 330 may include a first light generator 332 for generating light in a first wavelength and a second light generator 334 for generating light in a second wavelength. In some examples, the first light generator 332 is a white light generator, which may be comprised of multiple discrete light generation components (e.g., multiple LEDs of different colors), and the second light generator 334 is a fluorescence excitation light generator, such as a laser diode.

The light source 330 includes a controller 336 for controlling light output of the light generators. The controller 336 may be configured to provide pulse width modulation (PWM) of the light generators for modulating intensity of light provided by the light source 330, which can be used to manage overexposure and underexposure. In some examples, nominal current and/or voltage of each light generator remains constant, and the light intensity is modulated by switching the light generators (e.g., LEDs) on and off according to a PWM control signal. In some examples, a PWM control signal is provided by the imaging controller 336. This control signal can be a waveform that corresponds to the desired pulse width modulated operation of light generators.

The imaging controller 320 may be configured to determine the illumination intensity required of the light source 330 and may generate a PWM signal that is communicated to the light source 330. In some examples, depending on the amount of light received at the sensor 304 and the integration times, the light source may be pulsed at different rates to alter the intensity of illumination light at the target scene. The imaging controller 320 may determine a required illumination light intensity for a subsequent frame based on an amount of light received at the sensor 304 in a current frame and/or one or more previous frames. In some examples, the imaging controller 320 is capable of controlling pixel intensities via PWM of the light source 330 (to increase/decrease the amount of light at the pixels), via operation of the shutter 312 (to increase/decrease the amount of light at the pixels), and/or via changes in gain (to increase/decrease sensitivity of the pixels to received light). In some examples, the imaging controller 320 primarily uses PWM of the illumination source for controlling pixel intensities while holding the shutter open (or at least not operating the shutter) and maintaining gain levels. The controller 320 may operate the shutter 312 and/or modify the gain in the event that the light intensity is at a maximum or minimum and further adjustment is needed.

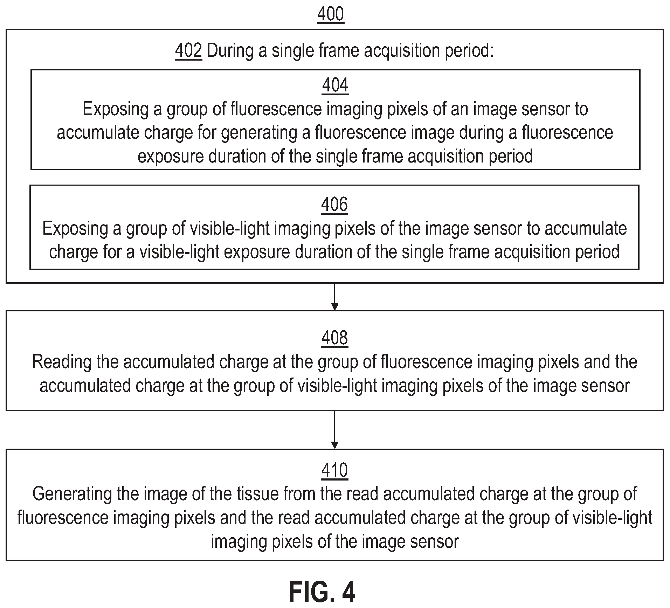

FIG. 4 provides an exemplary method 400 for generating an image of a tissue of a subject, in accordance with some examples. The method 400 may be performed by an exemplary imaging system. As described herein, the imaging system can illuminate the tissue of the subject using one or more fluorescence excitation illumination sources, one or more visible-light illumination sources, and/or other types of illumination sources, to accumulate charges at a plurality of rows of pixels of an image sensor. In some examples, the image sensor is a Bayer sensor. In some examples, the image senor is one of a plurality of image sensors of a multi-sensor imaging system such as a Philips prism. In some examples, the image sensor is part of an endoscopic imager, an open-field imager, or a flexible and/or chip-on-tip scope. In some examples, the imaging system is the imaging system 300 of FIG. 3, which has an imager (e.g., global shutter image sensor 302 of system 300) and a light source (e.g., light source 330 of system 300) that can comprise a fluorescence excitation illumination source and/or a visible-light illumination source.

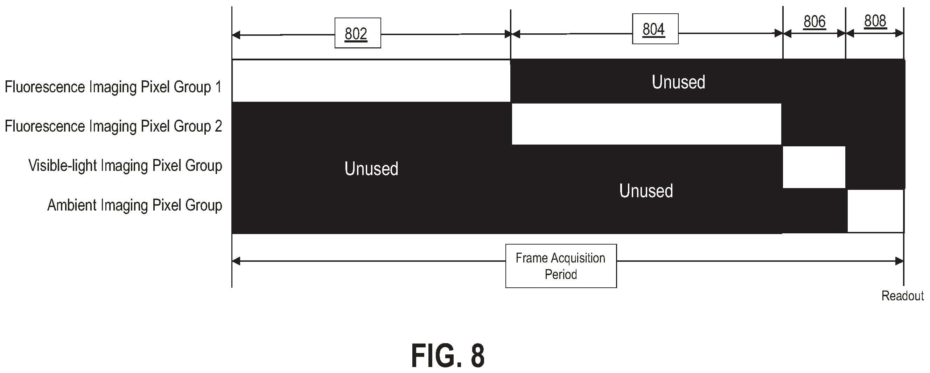

With reference to FIG. 4, block 402 comprises block 404 and block 406, which can occur during a single frame acquisition period (i.e., the same frame acquisition period). The single frame acquisition period can comprise a fluorescence exposure duration and a visible-light exposure duration, which may not overlap with each other. In the fluorescence exposure duration, the tissue is illuminated with a fluorescence excitation illumination source. In the visible-light exposure duration, the tissue is illuminated with a visible-light illumination source. The visible-light illumination source may be ambient illumination, a bright-light illumination source, or a combination thereof. In some examples, the tissue is illuminated with both ambient illumination and a bright-light illumination source during the visible-light exposure duration, as described in more detail with reference to FIG. 8.

At block 404, during the fluorescence exposure duration of the single frame acquisition period, an exemplary imaging system can expose a group of fluorescence imaging pixels of an image sensor to accumulate charge. At block 406, during the visible-light exposure duration of the single frame acquisition period, the imaging system can expose a group of visible-light imaging pixels of the image sensor to accumulate charge.

As described herein, "fluorescence imaging pixels" can refer to one or more pixels of the image sensor that are designated (e.g., based on a user input) to be exposed to generate fluorescence imaging data for a given frame acquisition period, and "visible-light imaging pixels" can refer to one or more pixels of the image sensor that are designated (e.g., based on a user input) to be exposed to generate visible-light image data for the given frame acquisition period. For the given frame acquisition period, the fluorescence imaging pixels and the visible-light imaging pixels belong to the same imaging sensor and may not overlap with each other. The designation of a given pixel may change across different frame acquisition periods. For example, the same pixel of the image sensor may be designated as a fluorescence imaging pixel for a first frame acquisition period and designated as a visible-light imaging pixel for a second frame acquisition period.

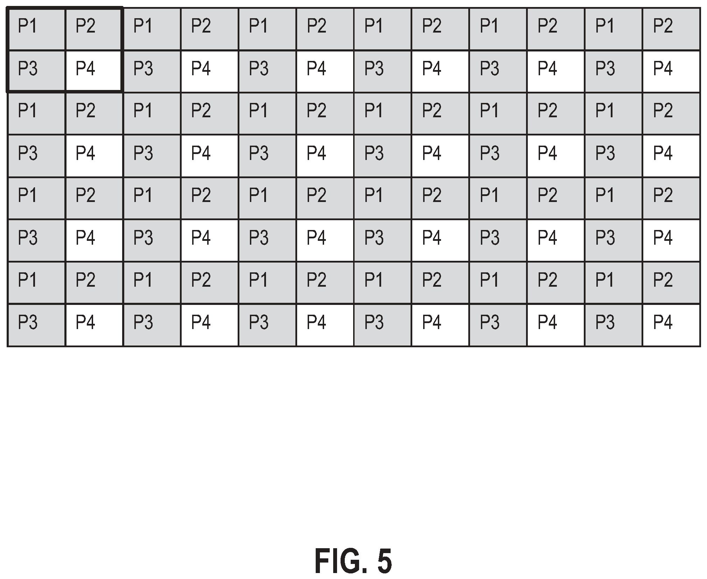

In some examples, an image sensor comprises a plurality of pixels divided into a plurality of pixel arrays, and one or more pixels in each pixel array can be designated as fluorescence imaging pixels and one or more other pixels in the pixel array can be designated as visible-light imaging pixels. FIG. 5 illustrates an exemplary pixel designation scheme for an image sensor for a given frame acquisition period, in accordance with some examples. With reference to FIG. 5, the image sensor comprises a plurality of pixels, specifically, a 12-by-8 grid of pixels. In the designation scheme, the plurality of pixels is divided into a plurality of 2-by-2 arrays. Each 2-by-2 array comprises four pixels, P1, P2, P3, and P4, corresponding to a Bayer pattern. Accordingly, there are 24 2-by-2 arrays in the image sensor. Further, P1, P2, and P3 in each pixel array are designated as fluorescence imaging pixels, and P4 in each pixel array is designated as a visible-light imaging pixel. In other words, the group of fluorescence imaging pixels comprises 3 pixels from each 2x2 pixel array of the plurality of 2x2 pixel arrays, and the group of bright-light imaging pixels comprises one pixel from each 2x2 pixel array of the plurality of 2x2 pixel arrays. The group of fluorescence imaging pixels can be configured to have a common fluorescence exposure period within a given frame acquisition period, and the group of visible-light imaging pixels can be configured to have a common visible-light exposure period. It should be appreciated that any type of sensor (with or without pixel arrays shown in FIG. 5) that has pixels with independent exposure control can operate according to the techniques described herein.

Returning to FIG. 4, at block 408, the imaging system can read the accumulated charge at the group of fluorescence imaging pixels and the accumulated charge at the group of visible-light imaging pixels of the image sensor. The system can generate a fluorescence image based on the read accumulated charge at the group of fluorescence imaging pixels. At block 410, the imaging system can generate the image of the tissue from the read accumulated charge at the group of fluorescence imaging pixels and the read accumulated charge at the group of visible-light imaging pixels of the image sensor.

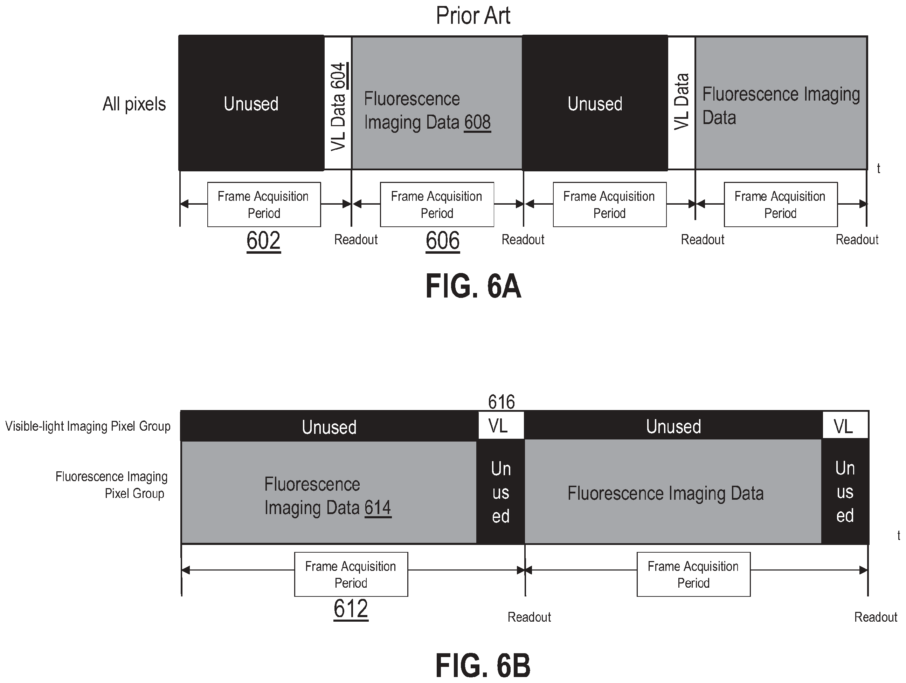

In some examples, the image generated in block 410 may comprise a brightness adjusted fluorescence image. Because the amplitude of the fluorescence imaging data is based on how much fluorophore is in the tissue rather than ambient brightness, the system may determine a brightness level based on the read accumulated charge at the group of visible-light imaging pixels of the image sensor and determine a gain setting for adjusting the fluorescence image based on the brightness level. In some examples, the system may execute an auto-gain-exposure algorithm to achieve a well illuminated image. The algorithm can calculate the average pixel value from the visible-light imaging pixels and then adjust exposure and gain to achieve a desired average pixel value. In some examples, the fluorescence gain is adjusted with a fixed scale from the pixel values from the visible-light imaging pixels. In some examples, the brightness information (e.g., derived from the pixel values of the visible-light imaging pixels) can be used to automatically calculate and set the exposure time for the fluorescence imaging pixels in the next frame acquisition period (e.g., by an auto-gain-exposure algorithm). Additional details for generating the brightness adjusted fluorescence image are provided in FIG. 6B below.

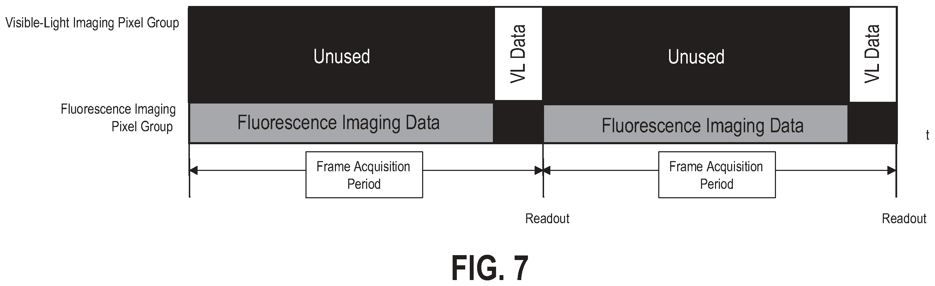

In some examples, the image generated in block 410 may comprise an overlay image. For example, the system may generate a visible-light image of the tissue from the read accumulated charge at the group of visible-light imaging pixels of the image sensor. The system may generate an overlay image based on the fluorescence image and the visible-light image of the tissue. Additional details for generating the overlay image are provided in FIGS. 7A-B below. In some examples, the image generated in block 410 may comprise an overlay image that comprises both a brightness adjusted fluorescence image and a visible-light image.

Because not all pixels of the image sensor are designated as fluorescence imaging pixels for a frame acquisition period, the resulting fluorescence image has a lower resolution than a fluorescence image generated by exposing all pixels of the image sensor. In some examples, the pixels designated as fluorescence imaging pixels and the pixels designated as visible-light imaging pixels can be alternated. Accordingly, resolution can be regained by averaging or otherwise combining the multiple frames. In the example in FIG. 5, for one frame acquisition period, P1 to P3 in each pixel array can be designated as fluorescence imaging pixels; for the next frame acquisition period, P4 in each pixel array can be designated as a fluorescence imaging pixel. The fluorescence imaging data from the two frame acquisition periods may be combined to generate a full-resolution fluorescence image frame.

In some examples, the image sensor can comprise additional groups of pixels for obtaining additional types of imaging data during the single frame acquisition period. For example, some pixels on the image sensor can be designated as ambient imaging pixels. "Ambient imaging pixels" can refer to one or more pixels of the image sensor that are designated (e.g., based on a user input) to be exposed to generate ambient light imaging data. The imaging system can expose, during the single frame acquisition period, the group of ambient imaging pixels of the image sensor to accumulate charge for an ambient light exposure duration while the tissue is not illuminated by the bright-light illumination source or the fluorescence excitation illumination source. The imaging system can read the accumulated charge at the group of ambient imaging pixels of the image sensor and determine an ambient brightness level based on the read accumulated charge at the group of ambient imaging pixels. The ambient brightness can be used to adjust the fluorescence image frame and/or the visible-light image frame. For example, if the gain and exposure time is the same for the ambient imaging pixels and the fluorescence imaging pixels, the ambient brightness may be subtracted from the fluorescence image. In some examples, additional noise filtering and/or scaling may be performed on the ambient brightness prior to subtraction.

As another example, the group of fluorescence imaging pixels in block 404 is a first group of fluorescence imaging pixels and the tissue is illuminated using a first fluorescence excitation illumination source corresponding to a first fluorophore in block 404. In addition, some other pixels on the image sensor can be designated as a second group of fluorescence imaging pixels. The system can expose, during the single frame acquisition period, the second group of fluorescence imaging pixels of the image sensor to accumulate charge for a second fluorescence exposure duration while the tissue is illuminated by a second fluorescence excitation illumination source corresponding to a second fluorophore. The system can read the accumulated charge at the second group of fluorescence imaging pixels of the image sensor and generate a second fluorescence image based on the read accumulated charge at the second group of fluorescence imaging pixels of the image sensor. The second fluorescence image can be similarly adjusted based on the brightness level to generate a second brightness adjusted fluorescence image, and/or can be used to generate an overlay image.

FIGS. 6A-B illustrate an exemplary comparison between a prior art imaging system and an exemplary imaging system of the present disclosure, in accordance with some examples. FIG. 6A illustrates an exposure timing diagram for the prior art imaging system. As shown in FIG. 6A, all pixels of the image sensor have the same exposure configuration. In a first frame acquisition period 602, all pixels of the image sensor are unused for most of the frame acquisition period, and then exposed to obtain visible-light imaging data 604 in a second portion of the frame acquisition period. In the next frame acquisition period 606, all pixels are exposed during the entire frame period to obtain fluorescence imaging data 608. The system may then generate an image based on the visible-light imaging data 604 and the fluorescence imaging data 608. The image may be a brightness adjusted fluorescence image, an overlay image, or the like. The prior art imaging system in FIG. 6A is undesirable because generating the final image takes two frame periods 602 and 606, but the frame period 602 is not used to obtain fluorescence imaging data at all but rather to obtain visible-light imaging data. Further, much of the frame period 602 is unused. In other words, the system needs to forego half of the total exposure time in order to obtain visible-light imaging data, much of which may go unused, at the expense of the potential exposure time for the fluorescence imaging data, thus resulting in suboptimal fluorescence imaging data.

In FIG. 6B, for a single frame acquisition period 612, some pixels of the image sensor (i.e., the fluorescence imaging pixel group) can be exposed to obtain fluorescence imaging data and other pixels of the image sensor (i.e., the visible-light imaging pixel group) can be exposed to obtain visible-light imaging data. Specifically, for the first (and longer) portion of the frame acquisition period 612, the system can expose the group of fluorescence imaging pixels to accumulate charge to obtain fluorescence imaging data 614. Then for the second (and shorter) portion of the frame acquisition period 612, the system can expose the group of visible-light imaging pixels of the image sensor to accumulate charge to obtain visible-light imaging data 616. At readout, the system can read the accumulated charge at the group of fluorescence imaging pixels and the accumulated charge at the group of visible-light imaging pixels of the image sensor. The system may then generate an image based on the visible-light imaging data 616 and the fluorescence imaging data 614. The image may be a brightness adjusted fluorescence image, an overlay image, or the like.

In FIG. 6B, more pixels of the image sensor are designated as fluorescence imaging pixels than visible-light imaging pixels. For example, as shown in FIG. 5, 3 pixels (P1 to P3) in each 2-by-2 pixel array can be designated as fluorescence imaging pixels while one pixel (P4) can be designated as a visible-light imaging pixel. Accordingly, more of the pixels of the image sensor are used to obtain fluorescence imaging data, resulting in a richer and higher-resolution fluorescence image than the corresponding visible-light image of the same frame. This is particularly advantageous because fluorescence signals may be relatively weak. Meanwhile, few of the pixels of the image sensor are used to obtain visible-light imaging data, because the visible-light imaging data may be used to determine the brightness level for auto gain adjustment and thus higher resolution might not be necessary.

The advantages of the pixel-specific exposure scheme are evident by comparing the two timing diagrams in FIGS. 6A and 6B. In FIG. 6A, the system alternates between a visible-light image frame period and a fluorescence image frame period, with all pixels of the image sensor exposed together. Image sensors are usually constrained with the speed of the data interface. With any given data rate, the maximum exposure time of all pixels is defined. In the depicted example, the frame acquisition period 612 equals the sum of the two frame acquisition periods 602 and 606. Thus, in both timing diagrams, a final image is generated in the same amount of time. However, the fluorescence imaging data 614 in FIG. 6B is the result of a much longer exposure duration than the fluorescence imaging data 608 in FIG. 6A and the visible-light imaging data 604 and 616 are generated using the same amount of exposure time. Thus, the pixel-specific exposure scheme in FIG. 6B results in higher-quality fluorescence imaging data (e.g., better signal-to-noise ratio) without compromising frame rate and while still acquiring visible-light imaging data. Furthermore, the scheme in FIG. 6B requires only half of the data transmission rate of the scheme of FIG. 6A.

FIG. 7 illustrates another exemplary imaging system, in accordance with some examples. Comparing to FIG. 6B, more pixels of the image sensor are designated in FIG. 7 as visible-light imaging pixels than fluorescence imaging pixels. For example, with reference to FIG. 5, 3 pixels (P1 to P3) in each 2-by-2 pixel array can be designated as visible-light imaging pixels while one pixel (P4) can be designated as a fluorescence imaging pixel. In one particular implementation, the red, a first green, and blue pixels in each pixel array are designated to generate visible-light imaging data while the second green pixel in the pixel array is designated to generate fluorescence imaging data. Comparing to FIG. 6B, the exposure scheme in FIG. 7 can generate higher-resolution visible-light imaging data. This can be advantageous when the visible-light imaging data is used to generate a visible-light image in the overlay mode rather than to only obtain brightness information for auto gain adjustment.

FIG. 8 illustrates another exemplary imaging system, in accordance with some examples. In FIG. 8, the pixels of the image sensor are divided into four groups: a first group of fluorescence imaging pixels, a second group of fluorescence imaging pixels, a group of visible-light imaging pixels, and a group of ambient imaging pixels. In the depicted example, during a single frame acquisition period, the imaging system can expose the first group of fluorescence imaging pixels in duration 802 (when the tissue is illuminated with a first fluorescence excitation illumination source corresponding to a first fluorophore and potentially ambient illumination), expose the second group of fluorescence imaging pixels in duration 804 (when the tissue is illuminated with a second fluorescence excitation illumination source corresponding to a second fluorophore and potentially ambient illumination), expose the group of visible-light imaging pixels in duration 806 (when the tissue is illuminated with a bright-light illumination source and potentially ambient illumination), and expose the group of ambient imaging pixels in duration 808 (when all other illumination sources are off other than potential ambient illumination). At readout, the imaging system can read the accumulated charge at each group of pixels to generate first fluorescence imaging data, second fluorescence imaging data, visible-light imaging data, and ambient imaging data. Various images can be generated, including but not limited to: a fluorescence image corresponding to the first fluorophore, a brightness adjusted or ambient subtracted version thereof, a fluorescence image corresponding to the second fluorophore, a brightness adjusted or ambient subtracted version thereof, a visible-light image, an ambient subtracted version thereof, or any combination thereof. The depicted approach can provide higher framerate acquisitions - in a single frame period, multiple types of fluorescence images and/or ambient illumination data can be obtained.

In some examples, the image sensor comprises a plurality of pixel arrays, each pixel array being a Bayer RGGB 2x2 array. The imaging system may apply different timing for the two G pixels. Green is 70% of luminance, and it has been demonstrated that creating a luminance based on G only is feasible. Thus, the system can set two different exposure durations for the two G pixels, which results in a larger dynamic range. In case of a multi-sensor imaging system (e.g., a Philips prism), all 2x2 pixels can be used for the HDR generation. The same pixels can be used for each of the sensors. For example, two pixels can be used for shorter exposure and 2 pixels can be used for longer exposure. As another example, two pixels can be used for medium exposure, one pixel can be used for longer exposure, and one pixel can be used for shorter exposure. In some examples, the image sensor comprises an RGBIR sensor.

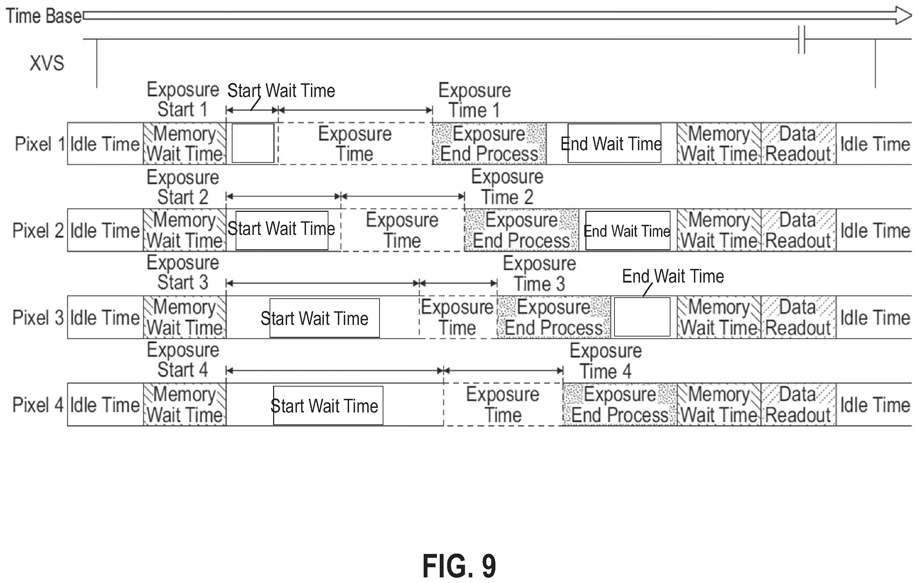

The exposure time for the group of fluorescence imaging pixels and the group of visible-light imaging pixels can be set by user inputs or automatically. FIG. 9 illustrates an exemplary configuration of exposure time for each pixel in a 2-by-2 pixel array. Each pixel in the pixel array can be configured to have a specific exposure timing (e.g., the duration of the exposure, the start of the exposure, etc.). In the depicted example in FIG. 9, for each of pixels P1 to P4, the time period marked "Exposure Time" is the exposure period. The user can specify the "Start Wait Time" duration and the "End Wait Time" duration, which in turn define the start time and duration of the exposure period for each pixel. The same timing scheme can apply to each pixel array in the image sensor. It should be appreciated that the mechanism for setting pixel-wise exposure period shown in FIG. 9 is merely exemplary. In some examples, the exposure time for each pixel in the image sensor can be set individually.

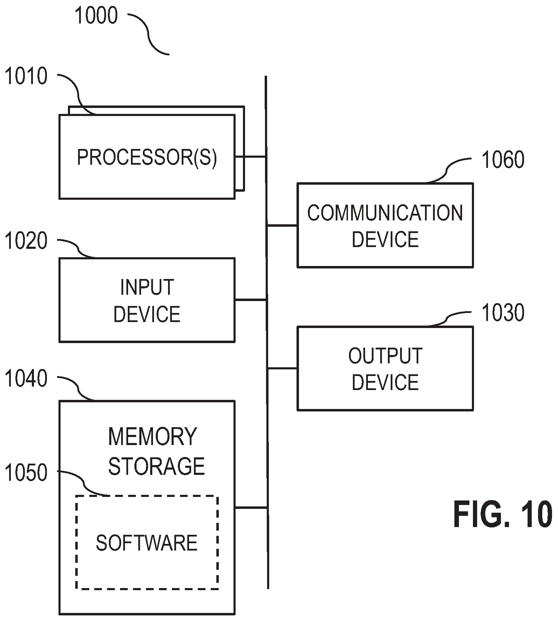

The operations described above are optionally implemented by components depicted in FIG. 10. FIG. 10 illustrates an example of a computing device in accordance with one embodiment. Device 1000 can be a host computer connected to a network. Device 1000 can be a client computer or a server. As shown in FIG. 10, device 1000 can be any suitable type of microprocessor-based device, such as a personal computer, workstation, server or handheld computing device (portable electronic device) such as a phone or tablet. The device can include, for example, one or more of processor 1010, input device 1020, output device 1030, storage 1040, and communication device 1060. Input device 1020 and output device 1030 can generally correspond to those described above, and can either be connectable or integrated with the computer.

Input device 1020 can be any suitable device that provides input, such as a touch screen, keyboard or keypad, mouse, or voice-recognition device. Output device 1030 can be any suitable device that provides output, such as a touch screen, haptics device, or speaker.

Storage 1040 can be any suitable device that provides storage, such as an electrical, magnetic or optical memory including a RAM, cache, hard drive, or removable storage disk. Communication device 1060 can include any suitable device capable of transmitting and receiving signals over a network, such as a network interface chip or device. The components of the computer can be connected in any suitable manner, such as via a physical bus or wirelessly.

Software 1050, which can be stored in storage 1040 and executed by processor 1010, can include, for example, the programming that embodies the functionality of the present disclosure (e.g., as embodied in the devices as described above).

Software 1050 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1040, that can contain or store programming for use by or in connection with an instruction execution system, apparatus, or device.

Software 1050 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

Device 1000 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Device 1000 can implement any operating system suitable for operating on the network. Software 1050 can be written in any suitable programming language, such as C, C++, Java or Python. In various embodiments, application software embodying the functionality of the present disclosure can be deployed in different configurations, such as in a client/server arrangement or through a Web browser as a Web-based application or Web service, for example.

The foregoing description, for the purpose of explanation, has been described with reference to specific examples. However, the illustrative discussions above are not intended to be exhaustive or to limit the invention to the precise forms disclosed. For the purpose of clarity and a concise description, features are described herein as part of the same or separate examples; however, it will be appreciated that the scope of the disclosure includes examples having combinations of all or some of the features described. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A method for generating an image of a tissue, comprising:
during a single frame acquisition period:
exposing a group of fluorescence imaging pixels of an image sensor to accumulate charge for generating a fluorescence image during a fluorescence exposure duration of the single frame acquisition period;
exposing a group of visible-light imaging pixels of the image sensor to accumulate charge for a visible-light exposure duration of the single frame acquisition period;
reading the accumulated charge at the group of fluorescence imaging pixels and the accumulated charge at the group of visible-light imaging pixels of the image sensor; and
generating the image of the tissue from the read accumulated charge at the group of fluorescence imaging pixels and the read accumulated charge at the group of visible-light imaging pixels of the image sensor.

2. The method of claim 1, wherein the tissue is illuminated with a fluorescence excitation illumination source during the fluorescence exposure duration.

3. The method of claim 1 or 2, wherein the tissue is illuminated with a visible-light illumination source during the visible-light exposure duration.

4. The method of claim 1, 2 or 3, wherein the image comprises a brightness adjusted fluorescence image, wherein generating the brightness adjusted fluorescence image comprises:
determining a brightness level based on the read accumulated charge at the group of visible-light imaging pixels of the image sensor;
determining a gain setting based on the brightness level;
generating a visible-light image of the tissue from the read accumulated charge at the group of bright-light imaging pixels of the image sensor; and
generating an overlay image based on the fluorescence image and the visible-light image of the tissue.

5. The method of any one of the preceding claims, wherein the fluorescence exposure duration and the visible-light exposure duration do not overlap.

6. The method of any one of the preceding claims, wherein the fluorescence exposure duration and the visible-light exposure duration are specified by one or more user inputs.

7. The method of any one of the preceding claims, wherein the fluorescence exposure duration is longer than the visible-light exposure duration.

8. The method of any one of the preceding claims, wherein the image sensor comprises a plurality of pixels divided into a plurality of pixel arrays, wherein the group of fluorescence imaging pixels comprises one or more pixels from each pixel array of the plurality of pixel arrays, and wherein the group of bright-light imaging pixels comprises one or more pixels from each pixel array of the plurality of pixel arrays.

9. The method of claim 8, wherein each pixel array of the plurality of pixel arrays is a 2x2 pixel array.

10. The method of claim 9, wherein the group of fluorescence imaging pixels comprises 3 pixels from each 2x2 pixel array of the plurality of 2x2 pixel arrays, and wherein the group of bright-light imaging pixels comprises one pixel from each 2x2 pixel array of the plurality of 2x2 pixel arrays.

11. The method of any one of the preceding claims, wherein the tissue is illuminated with ambient illumination and not a bright-light illumination source during the visible-light exposure duration.

12. The method of any of claims 1-10, wherein the tissue is illuminated with both ambient illumination and a bright-light illumination source during the visible-light exposure duration, the method further comprising:
exposing, during the single frame acquisition period, a group of ambient imaging pixels of the image sensor to accumulate charge for an ambient light exposure duration while the tissue is not illuminated by the bright-light illumination source or the fluorescence excitation illumination source;
reading the accumulated charge at the group of ambient imaging pixels of the image sensor; and
determining an ambient brightness level based on the read accumulated charge at the group of ambient imaging pixels of the image sensor.

13. The method of claim 2 or any of claims 3-12 as far as dependent from claim 2, wherein the fluorescence excitation illumination source is a first fluorescence excitation illumination source corresponding to a first fluorophore, wherein the fluorescence exposure duration is a first fluorescence exposure duration, and wherein the group of fluorescence imaging pixels is a first group of fluorescence imaging pixels, the method further comprising:
exposing, during the single frame acquisition period, a second group of fluorescence imaging pixels of the image sensor to accumulate charge for a second fluorescence exposure duration while the tissue is illuminated by a second fluorescence excitation illumination source corresponding to a second fluorophore;
reading the accumulated charge at the second group of fluorescence imaging pixels of the image sensor; and
generating a second fluorescence image based on the read accumulated charge at the second group of fluorescence imaging pixels of the image sensor.

14. The method of any one of the preceding claims, wherein the image sensor is part of an endoscopic imager, a flexible and/or chip-on-tip scope, or an open field imager.

15. A system for generating an image of a tissue, comprising an image sensor configured for:
during a single frame acquisition period:
exposing a group of fluorescence imaging pixels of the image sensor to accumulate charge for generating a fluorescence image during a fluorescence exposure duration of the single frame acquisition period;
exposing a group of visible-light imaging pixels of the image sensor to accumulate charge for a visible-light exposure duration of the single frame acquisition period;
reading the accumulated charge at the group of fluorescence imaging pixels and the accumulated charge at the group of visible-light imaging pixels of the image sensor; and
generating the image of the tissue from the read accumulated charge at the group of fluorescence imaging pixels and the read accumulated charge at the group of visible-light imaging pixels of the image sensor.
